# EUROPEAN PATENT SPECIFICATION

(11) **EP 3 603 482 B1**
(45) Date of publication and mention of the grant of the patent: **22.03.2023**
(21) Application number: 18777793.3
(22) Date of filing: 19.02.2018
(51) Int. Cl.: A61B 1/045, G02B 23/26, A61B 1/00

(54) **ENDOSCOPE SYSTEM, PROCESSOR DEVICE FOR OPERATING ENDOSCOPE SYSTEM**
ENDOSKOPISCHES SYSTEM, PROZESSORVORRICHTUNG ZUM BETRIEB EINES ENDOSKOPISCHEN SYSTEMS
SYSTÈME D'ENDOSCOPE, DISPOSITIF DE PROCESSEUR POUR L'ACTIONNEMENT DU SYSTÈME D'ENDOSCOPE

(30) Priority: 30.03.2017 JP 2017068084
(43) Date of publication of application: 05.02.2020
(73) Proprietor: FUJIFILM Corporation, Tokyo 106-8620 (JP)
(72) Inventor: WATANABE, Hiroki, Ashigarakami-gun Kanagawa 258-8538 (JP)
(74) Representative: Klunker IP Patentanwälte PartG mbB
(86) International application number: PCT/JP2018/005620
(87) International publication number: WO 2018/179986

(56) References cited:
- WO-A1-2016/199273
- JP-A- 2011 036 600
- JP-A- 2011 156 203
- JP-A- 2011 200 283
- JP-A- 2011 206 251
- JP-A- 2011 224 038
- US-A1- 2015 078 615
- US-A1- 2015 080 652
- US-B2- 8 681 208

## Description

### 1. Field of the Invention

The present invention relates to an endoscope system, a processor device, and a method of operating an endoscope system that detect a region to be paid attention to.

### 2. Description of the Related Art

In the medical field, endoscope systems comprising a light source device, an endoscope, and a processor device have been in widespread use. Particularly, in recent years, there are known endoscope systems that detect a region (hereinafter referred to as a region of interest) to be paid attention to using an image obtained by imaging an observation target and performs enhancement or the like as well as simply imaging the observation target using an endoscope. The region of interest is, for example, a region including a portion with the possibility of a lesion, or the like.

For example, an endoscope system of P2011-224038A detects a region of interest. Moreover, the endoscope system of P2011-224038A prevents overlooking of the region of interest by guessing a direction of the region of interest to display the direction in a case where the region of interest captured within the viewing field of the endoscope has disappeared.
US 2015/080652 A1 discloses an endoscope system, in which means are provided for identifying an area of interest as containing a possible lesion within the portion of images which are displayed on a display unit. Further, there is provided a tracking feature for tracking a location of the area of interest in relation to the endoscope.
JP 2011 036600 A discloses an endoscope system in which a region-of-interest is identified by virtual information constructed from information obtained by X-ray-imaging (CT imaging). It is not an endoscope image which is used to detect the region-of-interest.

### SUMMARY OF THE INVENTION

In endoscopy, it is important to find out a legion without missing the lesion. For this reason, the viewing field of an endoscope basically has a wide angle. For example, the viewing angle of an endoscope is usually at least about 140° to about 170°. In recent years, there is also an endoscope having a viewing angle exceeding this.

In a case where the endoscope has a wide angle, it is easy to capture a lesion within the viewing field of the endoscope. On the other hand, there is also a need to perform observation in a desired viewing field. That is, there is a demand for observing a specific range of an observation target with the same viewing field as a related-art familiar endoscope rather than observing the observation target in a wide range using the wide-angle endoscope.

An object of the invention is to provide an endoscope system, a processor device, and a method of operating an endoscope system that can easily capture a lesion and can display and observe a specific range.

An endoscope system of the invention comprises the features of claim 1.
Preferred embodiments are defined by the dependent claims.

A processor device usable in the endoscope system of the invention is defined by claim 10.

### BRIEF DESCRIPTION OF THE DRAWINGS

Fig. 1 is an external view of an endoscope system.
Fig. 2 is a block diagram of the endoscope system.
Fig. 3 is an explanatory view illustrating the viewing field of an endoscope.
Fig. 4 is a block diagram of an image processing unit.
Fig. 5 is a schematic view of an endoscope image to be acquired.
Fig. 6 is an explanatory view illustrating a relationship between the endoscope image to be acquired and a display range.
Fig. 7 is a schematic view of a display image.
Fig. 8 is an explanatory view illustrating a non-display region.
Fig. 9 is a display example of guidance information.
Fig. 10 is a flowchart illustrating the operation of the endoscope system.
Fig. 11 is an explanatory view of a case where a region of interest can be captured in a front viewing field.
Fig. 12 is an explanatory view of a case where the region of interest cannot be captured in the front viewing field.
Fig. 13 is an explanatory view of a case where the region of interest is captured in the non-display region.
Fig. 14 is another display example of the guidance information.
Fig. 15 is still another display example of the guidance information.
Fig. 16 is a display example of a case where the region of interest is detected in the display region.
Fig. 17 is an explanatory view of a case the region of interest disappears from the display region.
Fig. 18 is a block diagram of an image processing unit in a second embodiment.
Fig. 19 is a flowchart of the second embodiment.
Fig. 20 is a schematic view of a capsule endoscope.

### DESCRIPTION OF THE PREFERRED EMBODIMENTS

### [First Embodiment]

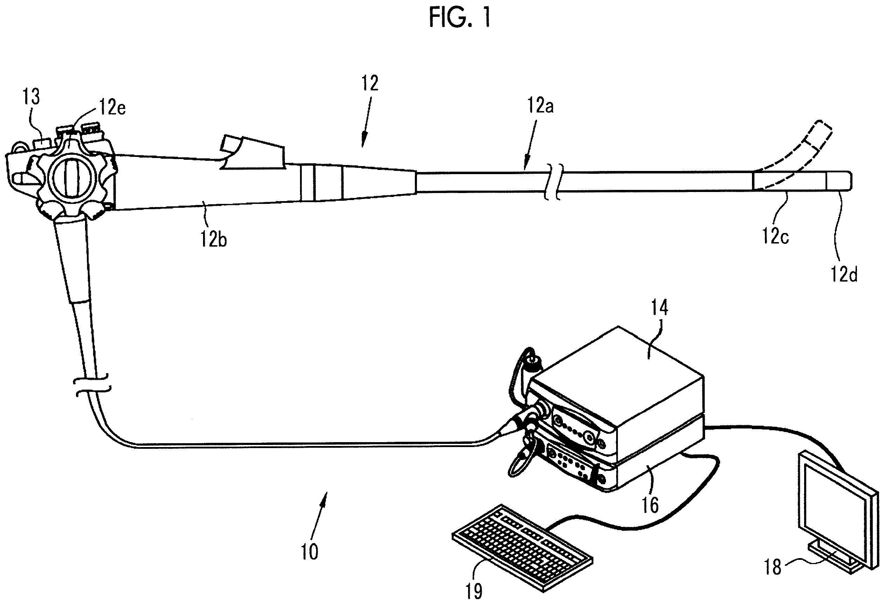

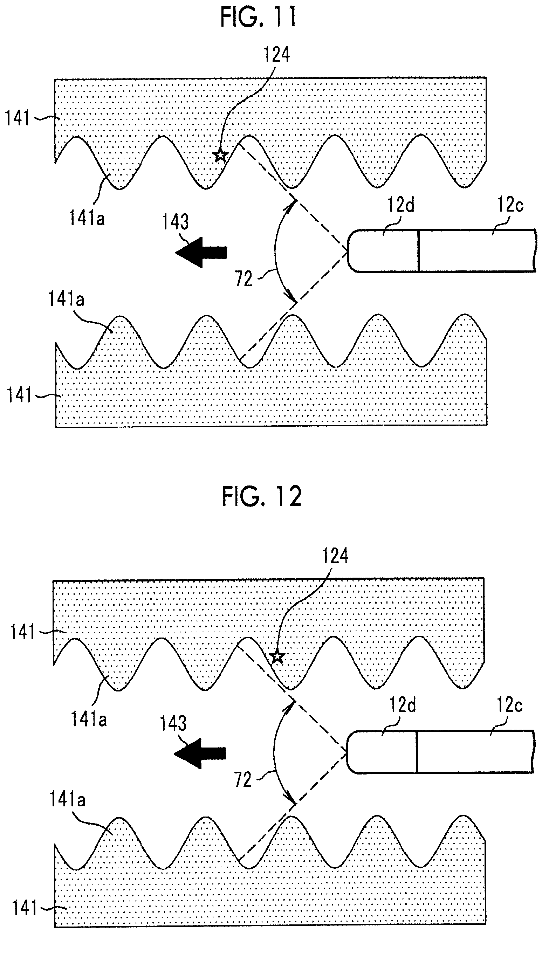

As illustrated in Fig. 1, an endoscope system 10 comprises an endoscope 12, a light source device 14, a processor device 16, a monitor 18, and a console 19. The endoscope 12 images an observation target 141 (refer to Fig. 11). The light source device 14 generates illumination light. The processor device 16 performs system control, image processing, and the like of the endoscope system 10. The monitor 18 is a display unit that displays at least a portion of an endoscope image. The console 19 is an input device that performs setting input and the like to the processor device 16 and the like.

The endoscope 12 has an insertion part 12a to be inserted into a subject, an operating part 12b provided at a proximal end portion of the insertion part 12a, a bending part 12c provided on a distal end side of the insertion part 12a, and a distal end part 12d. By operating an angle knob 12e of the operating part 12b, the bending part 12c is bent. As the bending part 12c is bent, the distal end part 12d is directed in a desired direction. In addition, the distal end part 12d is provided with a jet port (not illustrated) that jets air, water, or the like toward the observation target 141. Additionally, the operating part 12b is provided with a zoom operating part 13 in addition to the angle knob 12e. By operating the zoom operating part 13, the observation target 141 can be enlarged or reduced for imaging.

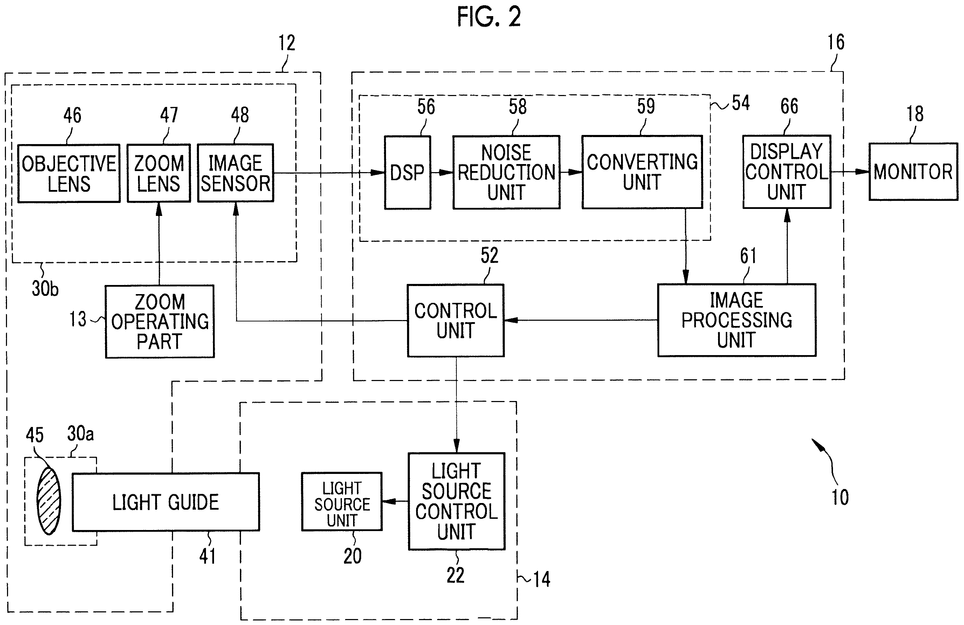

As illustrated in Fig. 2, the light source device 14 comprises a light source unit 20 that emits the illumination light, and a light source control unit 22 that controls driving of the light source unit 20.

The light source unit 20 comprises, for example, a plurality of light emitting diodes (LEDs) that emit light having different central wavelengths or wavelength ranges (hereinafter, simply referred to as having different wavelengths) as light sources, and a plurality of types of illumination light beams having different wavelengths can be emitted depending on light emission or turn-on of the respective LEDs, adjustment of light quantity, or the like. For example, the light source unit 20 is capable of emitting broadband purple light, blue light, green light, and red light with relatively wide wavelength ranges as the illumination light beams, respectively. Particularly, the light source unit 20 is capable of emitting narrowband (means that the wavelength range is a range of about 10 nm to 20 nm) purple light, blue light, green light, and red light as the illumination light beams, in addition to the broadband purple light, blue light, green light, and red light. More specifically, the light source unit 20 is capable of emitting narrowband purple light with a central wavelength of about 400 nm, first narrowband blue light with a central wavelength of about 450 nm, second narrowband blue light with a central wavelength of about 470 nm, narrowband green light with a central wavelength of about 540 nm, and narrowband red light with a central wavelength of about 640 nm, as the illumination light beams. In addition, the light source unit 20 is capable of emitting white light as an illumination light beam by combining the broadband or narrowband purple light, blue light, green light, and red light with each other.

In addition, instead of the LEDs, a combination of a laser diode (LD), a fluorescent body, and a band limiting filter, a combination of a lamp, such as a xenon lamp, and a band limiting filter, or the like can be used for the light source unit 20. It is natural that, even in a case where the LEDs constitute the light source unit 20, the fluorescent body or the band limiting filter can be used in combination with the LEDs.

The light source control unit 22 independently controls the timing of ON/OFF of the respective light sources that constitute the light source unit 20, the light emission amount thereof at the time of ON, and the like. As a result, the light source unit 20 is capable of emitting the plurality of types of illumination light beams with different wavelengths. Additionally, the light source control unit 22 controls the light source unit 20 in conformity with timing (so-called frame) for imaging of an image sensor 48.

The illumination light emitted from the light source unit 20 is incident on a light guide 41. The light guide 41 is built within the endoscope 12 and a universal cord, and propagates the illumination light up to the distal end part 12d of the endoscope 12. The universal cord is a cord that connects the endoscope 12, and the light source device 14 and the processor device 16 together. In addition, multi-mode fiber can be used as the light guide 41. As an example, a fine-diameter fiber cable of which the core diameter is 105 µm, the clad diameter is 125 µm, and a diameter including a protective layer serving as an outer cover is φ0. 3 to 0. 5 mm can be used.

The distal end part 12d of the endoscope 12 is provided with an illumination optical system 30a and an imaging optical system 30b. The illumination optical system 30a has an illumination lens 45, and emits the illumination light toward the observation target 141 via the illumination lens 45. The imaging optical system 30b has an objective lens 46, a zoom lens 47, and an image sensor 48. The image sensor 48 images the observation target 141, using reflected light or the like (including scattered light, fluorescence emitted from the observation target 141, fluorescence resulting from medicine administered to the observation target 141, or the like in addition to the reflected light) of the illumination light returning from the observation target 141 via the objective lens 46 and the zoom lens 47. The zoom lens 47 is moved by operating the zoom operating part 13, and enlarges or reduces the observation target 141 to be imaged using the image sensor 48.

The image sensor 48 is, for example, a color sensor having color filters of a primary color system, and comprises three types of pixels of a B pixel (blue pixel) having a blue color filter, a G pixel (green pixel) having a green color filter, and an R pixel (red pixel) having a red color filter. The blue color filter allows mainly purple to blue light to be transmitted therethrough. The green color filter allows mainly green light to be transmitted through. The red color filter allows mainly red light to be transmitted therethrough. In a case where the observation target 141 of the primary color system is imaged using the image sensor 48 as described above, three types of images including a B image (blue image) obtained from the B pixel, a G image (green image) obtained from the G pixel, and an R image (red image) obtained from the R pixel can be simultaneously obtained to the maximum.

In addition, as the image sensor 48, a charge coupled device (CCD) image sensor or a complementary metal-oxide semiconductor (CMOS) image sensor is available. Additionally, although the image sensor 48 of the present embodiment is the color sensor of the primary color system, a color sensor of a complementary color system can also be used. The color sensor of the complementary color system has, for example, a cyan pixel provided with a cyan color filter, a magenta pixel provided with a magenta color filter, a yellow pixel provided with a yellow color filter, and a green pixel provided with a green color filter. Images obtained from the above respective color pixels in a case where the color sensor of the complementary color system is used can be converted into the B image, the G image, and the R image in a case where complementary color-primary color conversion is performed. Additionally, instead of the color sensor, a monochrome sensor that is not provided with the color filters can be used as the image sensor 48. In this case, the above respective color images can be obtained by sequentially imaging the observation target 141, using the respective illumination light beams in colors, such as BGR.

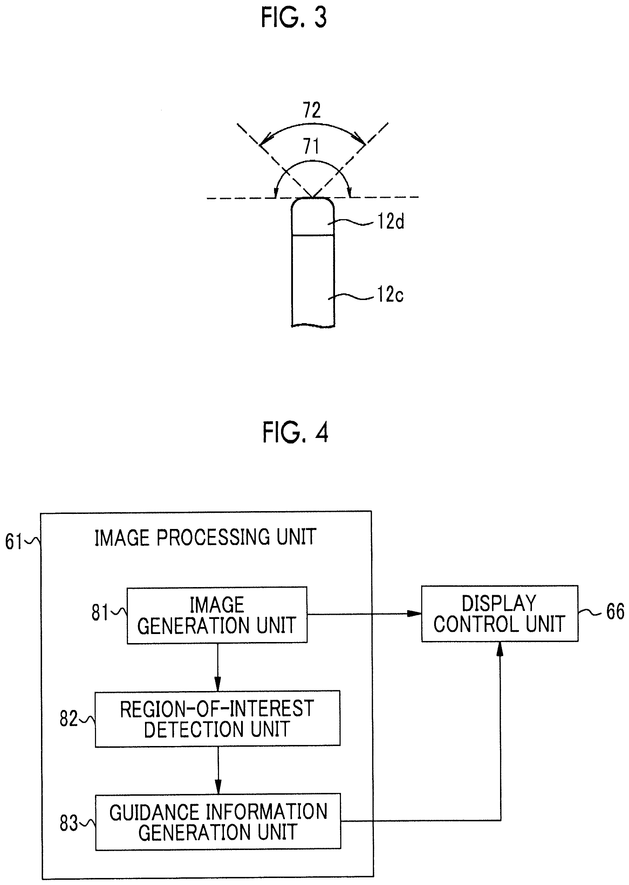

As illustrated in Fig. 3, the endoscope 12 has a so-called wide angle, and the angle (viewing angle) of a viewing field 71 (that is, the viewing field of the endoscope 12) of the imaging optical system 30b is about 140° or more. In the present specification, the wide angle means, for example, that the viewing angle is about 90° or more (preferably about 100° or more, and more preferably 120° or more). Hence, for example, an endoscope of which the viewing angle is 330°, an endoscope of which the viewing angle is 210°, and an endoscope of which the viewing angle is 230° to 240°, are all wide-angle endoscopes in the present specification, and each of these endoscopes can be suitably used as the endoscope 12 of the endoscope system 10. As long as the endoscope 12 have the wide angle, the observation target 141 that is substantially beside (the direction of a normal line on a side surface of the distal end part 12d) of the endoscope 12 or behind (a direction closer to a proximal side of the insertion part 12a than the normal line on the side surface of the distal end part 12d) thereof can be imaged in addition to the observation target 141 present in front of the endoscope 12 (a direction of a distal end surface of the distal end part 12d).

The illumination optical system 30a radiates uniform illumination light with substantially uniform illuminance and color at least in a range of the viewing field 71 of the imaging optical system 30b. For this reason, the endoscope 12 can preferably image the observation target 141 in a total range of the viewing field 71. In addition, although the endoscope system 10 images all the observation target 141 that falls within the viewing field 71, an image to be displayed on the monitor 18 for observation (diagnosis) is the observation target 141 present in the front viewing field 72. The front viewing field 72 is a partial imaging range including the front (front direction of the distal end part 12d in a case where the insertion part 12a is linearly extended) of the viewing field 71.

The processor device 16 has a control unit 52, an image acquisition unit 54, an image processing unit 61, and a display control unit 66 (refer to Fig. 2).

The control unit 52 performs overall control of the endoscope system 10, such as synchronous control between radiation timing of the illumination light and timing of the imaging. Additionally, in a case where input or the like of various settings is performed using the console 19 or the like, the control unit 52 inputs the settings to respective units of the endoscope system 10, such as the light source control unit 22, the image sensor 48, or the image processing unit 61.

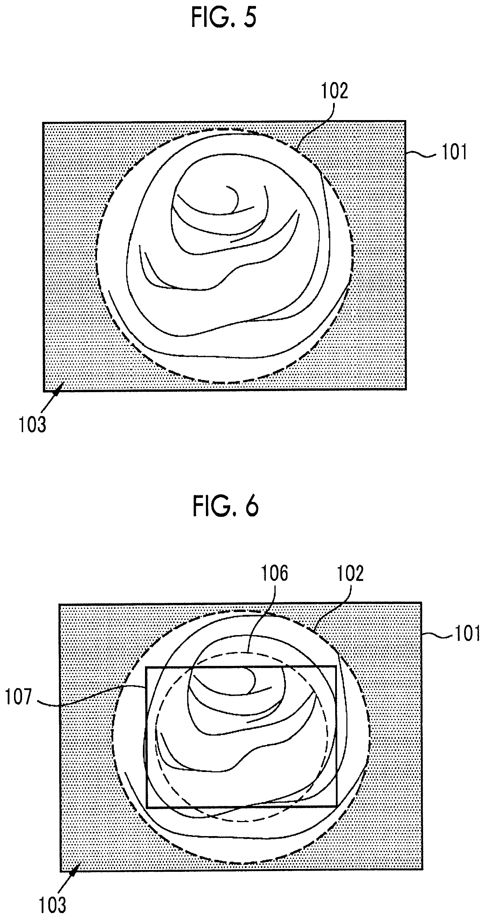

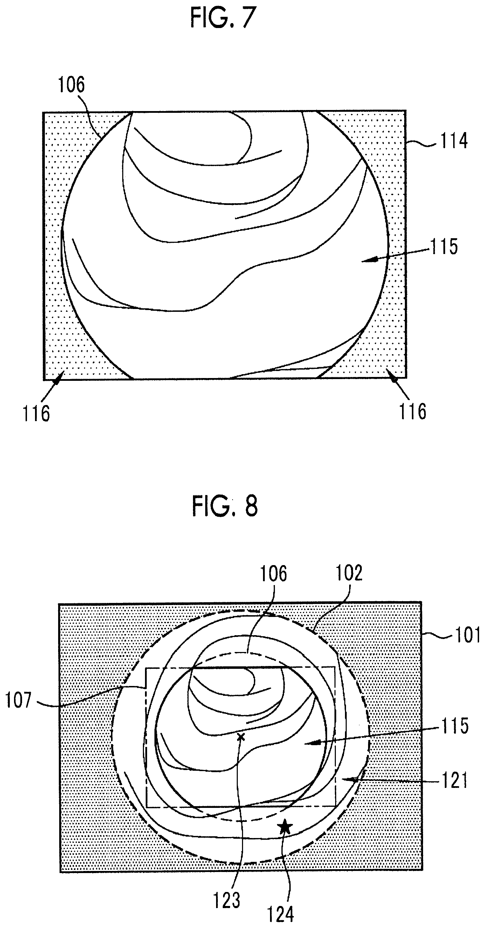

The image acquisition unit 54 acquires an image of the observation target 141 from the image sensor 48. In the present embodiment, the image sensor 48 has the color filters. Thus, the image acquisition unit 54 acquires an image for each illumination light beam and for each color filter. In addition, an image that the image acquisition unit 54 acquires from the image sensor 48, a display image generated by the image acquisition unit 54 using the image acquired from the image sensor 48, and an image that is intermediately generated using an image captured in order to generate the display image are all "endoscope images". Hereinafter, the term "image" simply means an endoscope image that is obtained by imaging the observation target 141 acquired by the image acquisition unit 54 from the image sensor 48. Additionally, the display endoscope image is referred to as a display image 114 (refer to Fig. 7), and the endoscope image that is intermediately generated is referred to as an intermediate image 101 (refer to Fig. 5). Additionally, since the endoscope 12 has the wide angle, at least the image obtained by imaging the observation target 141 using the endoscope 12, and the intermediate image 101 is an endoscope image of a wide angle obtained by imaging the observation target 141 that is present substantially beside or behind the endoscope 12 in addition to the observation target 141 present in front of the endoscope 12.

The image acquisition unit 54 has a digital signal processor (DSP) 56, a noise reduction unit 58, and a converting unit 59, and performs various kinds of processing on an acquired image, as needed, using these units.

The DSP 56 performs various kinds of processing, such as defect correction processing, offset processing, gain correction processing, linear matrix processing, gamma conversion processing, demosaicing processing, and YC conversion processing, on the acquired image, as needed.

The defect correction processing is the processing of correcting the pixel value of a pixel corresponding to a defective pixel of the image sensor 48. The offset processing is the processing of reducing a dark current component from the images subjected to the defect correction processing, and setting an accurate zero level. The gain correction processing is the processing of adjusting a signal level of each image by multiplying the images subjected to the offset processing by a gain. The linear matrix processing is the processing of enhancing color reproducibility on the images subjected to the offset processing, and the gamma conversion processing is the processing of adjusting the brightness and saturation of the images after the linear matrix processing. The demoisaicing processing (also referred to as equalization processing or synchronization processing) is the processing of interpolating the pixel value of a missing pixel, and is performed on the images after the gamma conversion processing. The missing pixel is a pixel with no pixel value due to the arrangement of the color filters (because other color pixels are disposed in the image sensor 48). For example, since the B image is an image obtained by imaging the observation target 141 in the B pixel, there is no pixel value in pixels at positions corresponding to the G pixel and the R pixel. In the demosaicing processing, the pixel values of the pixels at the positions of the G pixel and the R pixel of the image sensor 48 are generated by interpolating the B image. The YC conversion processing is the processing of converting the images after the demosaicing processing into a luminance channel Y, a color difference channel Cb, and a color difference channel Cr.

The noise reduction unit 58 performs noise reduction processing using, for example, a moving average method, a median filter method, or the like, on the luminance channel Y, the color difference channel Cb, and the color difference channel Cr. The converting unit 59 re-converts the luminance channel Y, the color difference channel Cb, and the color difference channel Cr after the noise reduction processing into images in respective colors of BGR.

The image processing unit 61 generates a display image 114 or the like, using the image acquired by the image acquisition unit 54. Additionally, the image processing unit 61 performs processing, such as region detection, and calculation or generation of required information, using the image acquired by the image acquisition unit 54 or the intermediate image 101. More specifically, as illustrated in Fig. 4, the image processing unit 61 comprises an image generation unit 81, a region-of-interest detection unit 82, and a guidance information generation unit 83.

The image generation unit 81 generates at least using the display image 114, using one or a plurality of images acquired by the image acquisition unit 54. In the present embodiment, the image generation unit 81 first generates the intermediate image 101 illustrated in Fig. 5, using one or a plurality of images acquired by the image acquisition unit 54. The intermediate image 101 is an endoscope image including the total range of the viewing field 71 of the endoscope 12. The imaging surface of the image sensor 48 is a quadrangular shape, and a region where the imaging optical system 30b forms the image of the observation target 141 present in the viewing field 71 is substantially circular. For this reason, in the intermediate image 101, a region (hereinafter referred to as a full viewing field region) 102 corresponding to the viewing field 71 is a portion of the intermediate image 101, and a blank region 103 where the observation target 141 is not reflected is present at an outer peripheral portion of the full viewing field region 102.

In a case where the intermediate image 101 is generated as described above, as illustrated in Fig. 6, the image generation unit 81 generates the display image 114 illustrated in Fig. 7 by extracting a quadrangular region (hereinafter referred to as an extraction range) 107 including most of the front viewing field region 106 corresponding to the front viewing field 72, in the intermediate images 101. That is, the image generation unit 81 generates the display image 114 by trimming the intermediate image 101 in the extraction range 107. In addition, in a case where the image generation unit 81 extracts the extraction range 107 from the intermediate image 101 to generate the display image 114, the image generation unit 81 does not display the region of the display image 114 outside the front viewing field region 106 with a mask 116 (for example, a black image). Accordingly, the display image 114 has the same appearance as an endoscope image to be obtained in the related-art endoscope system.

As compared to a case where the intermediate image 101 is displayed on the monitor 18 assuming that the size (the display range of the endoscope image) of a display screen of the monitor 18 is constant, the display image 114 is an endoscope image that is displayed after the front viewing field region 106 is substantially enlarged. For this reason, as a case where the intermediate image 101 is displayed on the monitor 18 is compared with a case where the display image 114 is displayed on the monitor 18, the observation target 141 captured to the front viewing field 72 is largely reflected in a case where the display image 114 is displayed on the monitor 18. As a result, in a case where a region to be paid attention to is present in the observation target 141 captured within the front viewing field 72, the region is also largely reflected.

In addition, in the present embodiment, the image generation unit 81 generates the display image 114 using the intermediate image 101. However, the image generation unit 81 can directly generate the display image 114, using one or a plurality of images acquired the image acquisition unit 54, without passing through the intermediate image 101. Additionally, in the present embodiment, in a case where the extraction range 107 is extracted from the intermediate image 101, the region of the display image 114 outside the front viewing field region 106 is not displayed by the mask 116. However, the mask processing can be omitted. In this case, the observation target 141 present outside the front viewing field region 106 is reflected in the portion of the mask 116 in the display image 114. For this reason, the display image 114 is, for example, a quadrangular endoscope image which includes the front viewing field region 106 and on which the observation target 141 is reflected in its entirety.

The region-of-interest detection unit 82 detects a region of interest 124 at least in a non-display region 121 out of a display region 115 that is that is a portion of the endoscope image and is to be displayed on the monitor 18 that is a display unit, and the non-display region 121 that is a portion of the endoscope image and is a portion excluding the display region 115 from the endoscope image. Hereinafter, the processing for detecting the region of interest 124 that the region-of-interest detection unit 82 executes in the non-display region 121 is referred to as a region of interest detection processing.

The endoscope image that the region-of-interest detection unit 82 uses for the detection of the region of interest 124 is one or a plurality of images acquired by the image acquisition unit 54 or the intermediate image 101 generated by the image generation unit 81. In the present embodiment, the region-of-interest detection unit 82 detects the region of interest 124, using the intermediate image 101.

The display region 115 is at least a region including the image of the observation target 141 present in front of the endoscope 12. Specifically, as illustrated in Fig. 8, the display region is a common portion between the front viewing field region 106 and the extraction range 107, in the intermediate image 101 (in a case where one or a plurality of images acquired by the image acquisition unit 54 is used, an image to be used among these images). However, in a case where the mask processing is not performed in the display image 114, the entire extraction range 107 is the display region 115.

The non-display region 121 is at least a region including the observation target 141 that is present substantially beside or behind the endoscope 12. Specifically, the non-display region is a remaining portion excluding the display region 115 from the full viewing field region 102 in the intermediate image 101 (in a case where one or a plurality of images acquired by the image acquisition unit 54 are used, an image to be used among these images). In the present embodiment, a substantially annular portion excluding a barrel-shaped display region 115 from the center of the circular full viewing field region 102 is the non-display region 121.

The region of interest 124 is a region to be paid attention to as a target of examination or diagnosis. The region of interest 124 is, for example, a region including a lesioned part represented by cancer, a benign tumor part, an inflammable part (including a portion with a change, such as bleeding or atrophy, in addition to the so-called inflammation), an ablation trace or coloring agent resulting from heating, a marking part marked by coloring resulting from a fluorescent agent or the like, or a biopsy-performed part in which a biological test (so-called a biopsy) is performed. That is, a region where detailed observation is required irrespective of the possibility of a lesion, such as a region including a lesion, a region with the possibility of a lesion, a region where a certain measure, such as a biopsy, is taken, or a dark region (a region where observation light does not reach easily due to an inner part of a fold (pleat) or and the back of the lumen) can be the region of interest 124.
In the endoscope system 10, the region-of-interest detection unit 82 detects a region, which includes at least any of the lesioned part, the benign tumor part, the inflammable part, the marking part, or a biopsy-performed part, as the region of interest 124.

The region-of-interest detection unit 82 detects the region of interest 124 on the basis of pixel values of pixels or the distribution of pixel values in the non-display region 121. The pixel values or the distribution of the pixel values represent, for example, the shape (such as global undulations or local depressions protuberances of a mucous membrane) of the observation target 141 reflected to the non-display region 121, color (color, such as chlorosis resulting from inflammation, bleeding, redness, or atrophy), the features of tissue (the thickness, depth, and density of blood vessels or combinations thereof), or the features (pit pattern or the like) of structure. In the present embodiment, although the region-of-interest detection unit 82 detects the region of interest 124 in the non-display region 121, the region-of-interest detection unit 82 can detect the region of interest 124 also in the display region 115 as necessary.

The guidance information generation unit 83 generates the guidance information to the region of interest 124 present in the non-display region 121 in a case where the region-of-interest detection unit 82 has detected the region of interest 124 in the non-display region 121. The guidance information is, for example, information including one or plurality of regions among the direction of the region of interest 124 in the non-display region 121, the distance or angle to the region of interest 124 present in the non-display region 121, or information indicating the operation time of the endoscope 12 taken to bring the region of interest 124 in the non-display region 121 present into the display region 115. The guidance information in the present embodiment includes information for simply calculating the above direction, distance, or angle, or the operation time in addition to the information that directly indicates the above direction, distance, or angle, or the operation time.

The direction of the region of interest 124 is, for example, information indicating a direction along a line that connects a reference point, such as a center 123 of the display region 115 to the region of interest 124 that is present in the non-display region 121, and the direction of the region of interest 124 present in the non-display region 121 with reference to the center 123 of the display region 115. In the present embodiment, although the direction of the region of interest 124 is a direction on an image, such as the intermediate image 101, a three-dimensional direction on an actual space where the observation target 141 is present may be the direction of the region of interest 124.

The distance of the region of interest 124 is, for example, a length in the actual space from the reference point, such as the center 123 of the display region 115, to the region of interest 124. The distance of the region of interest 124 can be calculated, for example, from a length on an image, such as the intermediate image 101. For this reason, the length on the image, such as the intermediate image 101 may be "the distance of the region of interest 124".

The angle of the region of interest 124 is an angle in the actual space of the region of interest 124 that is measured (including estimation) around a distal end of the distal end part 12d with reference to the central axis of the insertion part 12a. This angle is substantially equal to, for example, an angle at which the distal end part 12d is bent in order to cause the center 123 of the display region 115 to coincide with the region of interest 124 present in the non-display region 121.

The operation time of the endoscope 12 taken to bring the region of interest 124 present in the non-display region 121 in the display region 115 is a time required in order to bend or move the distal end part 12d in order to put the region of interest 124 within the display region 115 in the display region 115 to display the region of interest 124 on the monitor 18. The guidance information generation unit 83 calculates the operation time of the above endoscope 12, using at least the distance from the display region 115 to the region of interest 124 present in the non-display region 121. Additionally, the guidance information generation unit 83 can more accurately calculate the operation time of the endoscope 12 in a case where a speed (bending speed) at which the distal end part 12d is bent or a speed (movement speed) at which the distal end part 12d moves along the observation target 141 is used for the calculation of the operation time of the endoscope 12 in addition to the above distance.

In the present embodiment, the guidance information generation unit 83 generates at least guidance information including "the direction of the region of interest 124" present in the non-display region 121. Additionally, in the present embodiment, all the centers of the viewing field 71, the front viewing field 72, the front viewing field region 106, the full viewing field region 102, the intermediate image 101, the display image 114, the display region 115, and the non-display region 121 coincide with the center 123. However, even in a case where one or a plurality of centers among these are different, the direction, distance, or angle of the region of interest 124 present in the non-display region 121 can be determined by the same method as above.

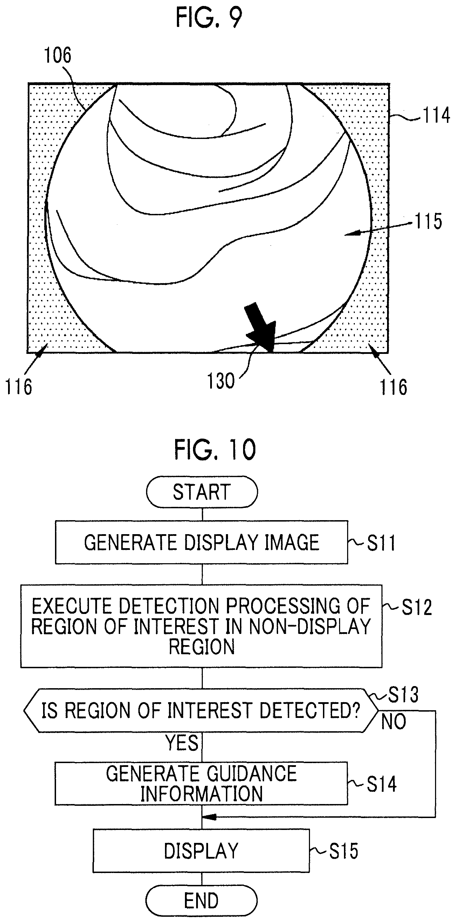

The display control unit 66 acquires the display image 114 from the image generation unit 81, converts the acquired endoscope image into a form suitable for display, and outputs the converted image to the monitor 18. Accordingly, the monitor 18 displays at least a portion of the endoscope image (display region 115). Additionally, the display control unit 66 acquires guidance information from the guidance information generation unit 83, and displays the guidance information on the monitor 18, which is the display unit, in addition to the display image 114 that is the endoscope image. As illustrated in Fig. 9, in the present embodiment, the display control unit 66 superimposes an arrow 130, which indicates the direction of the region of interest 124, on the display region 115 of the display image 114, and displays the superimposed image on the monitor 18.

Hereinafter, a flow of the operation of the endoscope system 10 will be described along the flowchart illustrated in Fig. 10. In a case where an observation is started, the endoscope system 10 images the observation target 141 irradiated with the illumination light, and consequently, the image acquisition unit 54 acquires an image from the image sensor 48. In a case where the image acquisition unit 54 acquires the image obtained by imaging the observation target 141, the image generation unit 81 generates the intermediate image 101 (refer to Fig. 5), and generates the display image 114 (refer to Fig. 7) (S11). Then, the region-of-interest detection unit 82 executes the detection processing of the region of interest 124 in the non-display region 121, using the intermediate image 101 (S12).

In a case where the region-of-interest detection unit 82 has detected the region of interest 124 in the non-display region 121 (S13: YES), the guidance information generation unit 83 generates the guidance information on the region of interest 124 of the non-display region 121. Specifically, the guidance information generation unit 83 generates information including at least the direction of the region of interest 124 as the guidance information. In a case where the guidance information generation unit 83 generates the guidance information, the display control unit 66 superimposes the arrow 130, which indicates the direction of the region of interest 124, on the display image 114, using the guidance information, and outputs the superimposed image to the monitor 18 (refer to Fig. 9). Accordingly, the monitor 18 displays the display image 114, and the arrow 130 that indicates the direction of the region of interest 124 superimposed on the display image 114 (S 15).

In a case where the region-of-interest detection unit 82 does not detect the region of interest 124 in the non-display region 121 (S13: NO), the guidance information generation unit 83 skips the generation of the guidance information. For this reason, the display control unit 66 displays the display image 114 acquired from the image generation unit 81 on the monitor 18 (S15).

As described above, in the endoscope system 10, the endoscope 12 has the wide-angle viewing field 71. However, the range of the front viewing field 72 that is a portion of the viewing field 71 is displayed on the monitor 18, using the display image 114. That is, according to the endoscope system 10, a specific range of the observation target can be observed and displayed as the same viewing field as the related-art familiar endoscope. Additionally, supposing that the size of the monitor 18 is determined, as compared to a case where the intermediate image 101 or the like including the entire viewing field 71 is displayed on the monitor 18 as it is, the observation target 141 in the front viewing field 72 is substantially enlarged and displayed. For this reason, the endoscope system 10 easily captures a lesion.

On the other hand, since the endoscope system 10 adopts the wide-angle endoscope 12, the endoscope system detects the region of interest 124 in the non-display region 121 that is not displayed due to the above display method. Then, in a case where the region of interest 124 is detected in the non-display region 121, the direction is displayed on the monitor 18 and the region of interest 124 is present in the non-display region 121. For this reason, an oversight of the region of interest 124 can be reduced compared to the related-art endoscope system with a relatively narrow viewing field. As a result, the endoscope system 10 captures a lesion more easily than the related-art endoscope system.

For example, as illustrated in Fig. 11, the observation target 141 is, for example, an esophagus, the stomach, intestines, or a trachea, or the like, and an inner surface of the observation target 141 (mucous membrane) is usually undulations (hereinafter referred to as folds (pleats)) 141a resulting from irregularities or a peristaltic motion. In a case where the region of interest 124 is present on the front side of the endoscope 12 of the fold 141a, the region of interest 124 can be captured in the front viewing field 72 while the endoscope 12 is moved back and forth in the insertion direction 143. In this case, in the monitor 18, the region of interest 124 can be visually recognized within the display region 115 similarly to the related-art endoscope system.

On the other hand, as illustrated in Fig. 12, in a case where the region of interest 124 is present on a back side (the surface of an inner part of the insertion direction 143 as seen from the endoscope 12) of the fold 141a, it is difficult to capture the region of interest 124 on the back side of the fold 141a in the front viewing field 72 that is narrow to the same extent as the related-art endoscope system unless the back side of each fold 141a is very carefully observed. For this reason, in a case where the related-art endoscope system, it is easy to overlook the region of interest 124 present on the back side of the fold 141a.

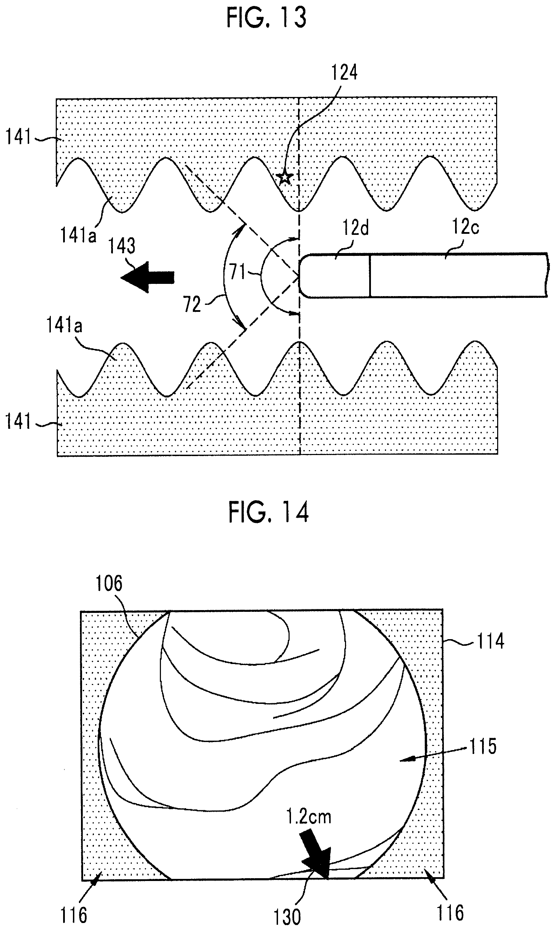

However, the endoscope system 10 practically adopts the wide-angle endoscope 12. Thus, as illustrated in Fig. 13, the region of interest 124 present on the back side of the fold 141a can be captured within the viewing field 71 while the endoscope 12 is relatively naturally inserted in the insertion direction 143. Here, in the endoscope system 10, a region outside the front viewing field 72 and inside the viewing field 71 is the non-display region 121. Thus, the region of interest 124 present on the back side of the fold 141a is not reflected on the monitor 18 in a state where the region of interest 124 on the back side of the fold 141a is captured outside the front viewing field 72 and inside the viewing field 71. For that reason, the endoscope system 10 urges observation or diagnosis of the region of interest 124 present on the back side of the fold 141a, using the guidance information indicating the direction of the region of interest 124 captured outside the front viewing field 72 and inside the viewing field 71. Hence, the endoscope system 10 can reduce the oversight of the region of interest 124 more than the related-art endoscope system.

Additionally, the related-art endoscope system, which uses the wide-angle endoscope, uses substantially the entirety of the viewing field thereof for display, and displays the guidance information, easily captures the region of interest 124 within the viewing field due to extending the viewing field of the endoscope. However, the observation target is reflected on a small scale, since an observation target is reflected on a small scale, the region of interest 124 may be overlooked as a result. In contrast, as described above, the endoscope system 10 makes the reduction of the oversight of the region of interest 124 in the display region 115, and the easy discovery of the region of interest 124 in which the wide-angle viewing field 71 is efficiently used compatible with each other. Thus, a lesion or the like is captured more than the above related-art endoscope system.

In addition to this, the display image 114 that the endoscope system 10 displays on the monitor 18 is substantially the same as that of an endoscope image that the related-art endoscope system with a relatively narrow viewing field displays, and is observable on one screen of the monitor 18 without a so-called joint. For this reason, the endoscope system 10 does not cause the oversight of the region of interest 124 resulting from the joint, and has no problems, such as an increase in a feeling of fatigue or difficulty in inspection as compared with a case where the observation target 141 should be observed with a plurality of screen or a plurality of images. Moreover, in a case where the wide-angle endoscope 12 is adopted and the entire viewing field 71 is used for display, it is difficult to perform treatment with high invasiveness, such as endoscopic resection due to the observation target 141 being relatively reduced. However, since the endoscope system 10 displays the display region 115 corresponding to the front viewing field 72 on the monitor 18, treatment with high invasiveness can also be performed similarly to the related art.

Moreover, in the endoscope system 10, the region-of-interest detection unit 82 detects the region of interest 124 present in the non-display region 121, using the intermediate image 101 that is one endoscope image, and the guidance information generation unit 83 generates the guidance information to the region of interest 124 present in the non-display region 121, using the intermediate image 101 that is one endoscope image. For this reason, as compared to the endoscope system that detects the region of interest 124 using a plurality of endoscope images that are sequentially obtained, or a case where the guidance information to the region of interest 124 present in the non-display region 121 is generated using a plurality of endoscope images that are sequentially obtained, the endoscope system 10 can particularly accurately obtain the detection and guidance information of the region of interest 124 in the viewpoint of simultaneity. A case where a plurality of images serving as a generation source of the intermediate image 101 are used instead of the intermediate image 101 is also the same as the above because one set of images that are simultaneously captured are used.

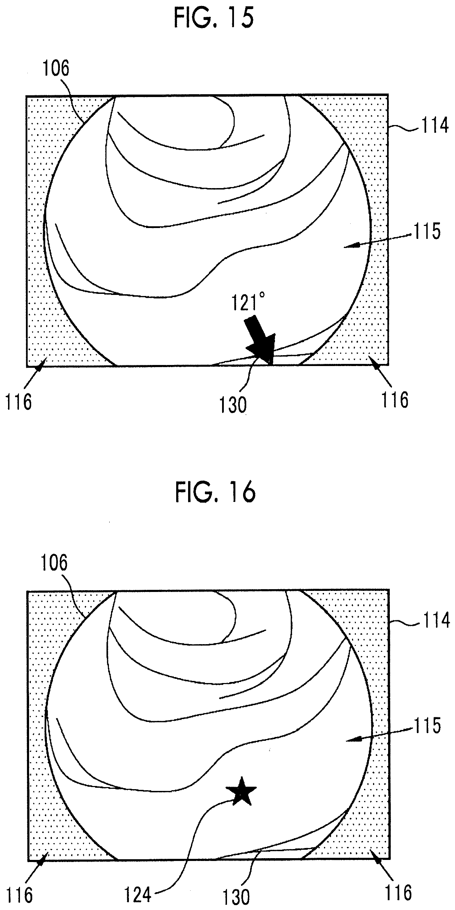

In the above first embodiment, the display control unit 66 displays the direction of the region of interest 124 on the monitor 18 as the guidance information. However, in addition to the direction of the region of interest 124 or instead of the direction of the region of interest 124, other guidance information can be displayed on the monitor 18. For example, as illustrated in Fig. 14, the display control unit 66 may display a distance ("1.2 cm" in Fig. 14) up to the region of interest 124 in addition to the arrow 130 indicating the direction of the region of interest 124. Additionally, as illustrated in Fig. 15, the display control unit 66 may display an angle ("121°" in Fig. 15) up to the region of interest 124 in addition to the arrow 130 indicating the direction of the region of interest 124. In this way, in a case where the distance, angle, or the like of the region of interest 124 is displayed in addition to the direction of the region of interest 124, guidance to the region of interest 124 can be more easily performed.

Additionally, the display of the distance is not limited to a notation by a numerical value like "1.2 cm" in Fig. 14. For example, the shape, number, thickness, length, or color of the arrow 130 may be a display corresponding to the distance. For example, it is possible to display the length of the arrow longer as the distance is longer, or it is possible to display the thickness of the arrow more largely as the distance is longer. The same applies to the display of the angle or the like.

In a case where the direction of the region of interest 124 is indicated as the guidance information, the display method thereof is not limited to the arrow 130 as long as the direction of the region of interest 124 can be known. For example, other optional aspects, such as a sentence (texts) or voice, can indicate the direction of the region of interest 124. The same applies to guidance information other than the direction. In addition, in a case where some or all of guidance information is shown by methods other than the display using the monitor 18, the entirety of the monitor 18, and a device for presenting some or all of the guidance information constitutes the display unit. For example, in a case where voice representing the guidance information is output using a loudspeaker (not illustrated), the monitor 18, and the loudspeaker constitute the display unit. For this reason, in the present specification, presentation of the guidance information using devices other than the monitor 18, such as the output of the voice representing the guidance information using a loudspeaker is included in the "presentation" of the guidance information.

In the first embodiment, the region-of-interest detection unit 82 detects the region of interest 124 in the non-display region 121. However, the region of interest 124 may be detected also in the display region 115 in addition to the non-display region 121. In this case, for example, as illustrated in Fig. 16, by enhancing the region of interest 124, which is detected in the display region 115, in the display image 114, a doctor or the like can more reliably recognize the presence of the region of interest 124.

### [Second Embodiment]

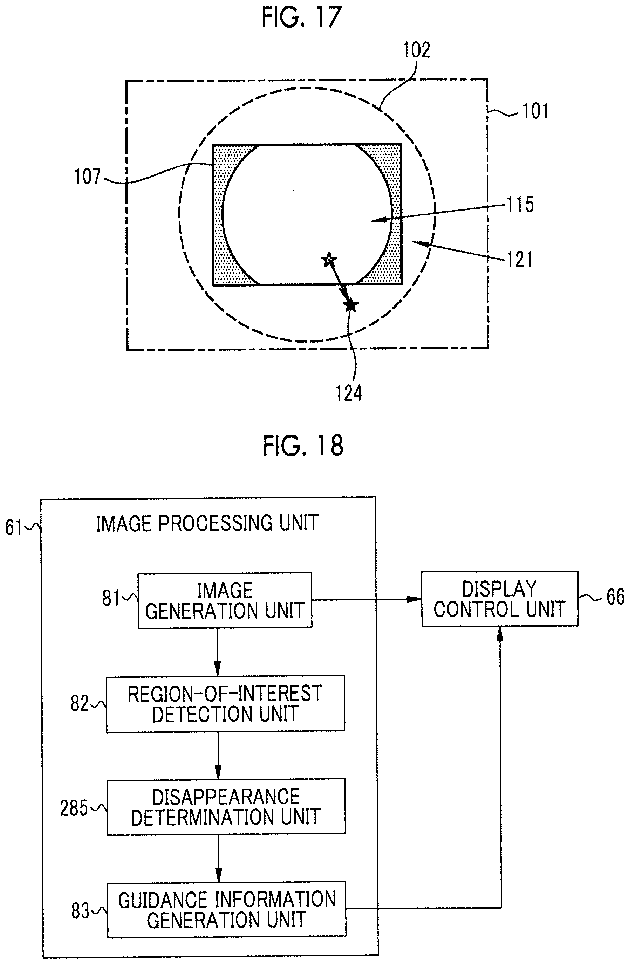

In the first embodiment, although the region-of-interest detection unit 82 detects the region of interest 124 in the non-display region 121, the region-of-interest detection unit 82 can detect the region of interest 124 also in the display region 115 in addition to the non-display region 121. Also, in a case where the region of interest 124 is detected also in the display region 115, as illustrated in Fig. 17, particularly, it is highly necessary to display the guidance information on the monitor 18 in a case where the region of interest 124 detected by the display region 115 has moved to the non-display region 121 due to the insertion and extraction or bending of the endoscope 12 and has disappeared from the display region 115.

As described above, in order to detect the disappearance of the region of interest from the display region 115, as illustrated in Fig. 18, the image processing unit 61 is provided with a disappearance determination unit 285 in addition to the image generation unit 81, the region-of-interest detection unit 82, and the guidance information generation unit 83. The disappearance determination unit 285 determines the disappearance of the region of interest from the display region 115 resulting from movement of the region of interest 124 detected in the display region 115 at a certain time to the non-display region 121 at a time after the certain time. Also, the guidance information generation unit 83 generates guidance information at least on the region of interest 124 determined to have disappeared from the display region 115 by the disappearance determination unit 285.

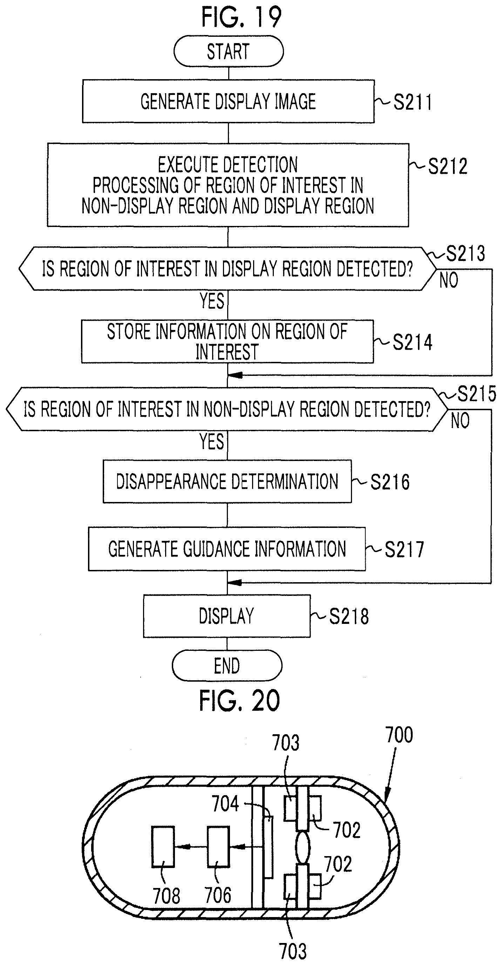

More specifically, the endoscope system 10 provided with the disappearance determination unit 285 operates as illustrated in Fig. 19. In a case where an observation is started, the endoscope system 10 images the observation target 141 irradiated with the illumination light, and consequently, the image acquisition unit 54 acquires an image from the image sensor 48. Then, the image generation unit 81 generates the intermediate image 101 (refer to Fig. 5), and generates the display image 114 (refer to Fig. 7) (S211). Then, the region-of-interest detection unit 82 executes the detection processing of the region of interest 124 in the non-display region 121 and the display region 115, using the intermediate image 101 (S212).

In a case where the region-of-interest detection unit 82 has detected the region of interest 124 in the display region 115, the disappearance determination unit 285 stores the information on the region of interest 124 of the display region 115 (S214). The information on the region of interest 124 of the display region 115 is, for example, features, such as the position, size and other shape of the region of interest 124 of the display region 115, a time (an imaging frame or the like) at which the region of interest 124 is detected in the display region 115, an endoscope image (the intermediate image 101 or the like) in a case where the region of interest 124 is detected, or combinations thereof.

Then, in a case where the region-of-interest detection unit 82 has detected the region of interest 124 in the non-display region 121, the disappearance determination unit 285 collates the information on the region of interest 124 detected in the display region 115 with the region of interest 124 of the non-display region 121, and determines whether or not the region of interest 124 detected in the display region 115 in the past is the same as the region of interest 124 detected in the non-display region 121 (the possibility that these regions are the same as each other is high) (S216). This is the disappearance determination performed by the disappearance determination unit 285.

In a case where the region of interest 124 detected in the display region 115 in the past is the same as the region of interest 124 detected in the non-display region 121 at a later time, the guidance information generation unit 83 moves from the display region 115 to the non-display region 121, and generates guidance information on the region of interest 124 that has disappeared from the display region 115. Then, the display control unit 66 superimposes the arrow 130, which indicates the direction of the region of interest 124, on the display image 114, using the guidance information, and outputs the superimposed image to the monitor 18. Accordingly, the monitor 18 displays the display image 114 and the arrow 130 or the like that indicates the direction of the region of interest 124 superimposed on the display image 114 (S218), and consequently, guides a doctor or the like to the region of interest 124 that has disappeared from the display region 115.

As described above, in a case where the region of interest 124 is detected also in the display region 115 and the guidance information is generated and displayed on the region of interest 124 that has disappeared from the display region 115, convenience is improved. Since the operation of the endoscope system 10 (endoscope 12) requires delicate and advanced art, it is common that region of interest 124 captured in the front viewing field 72 is missed out of the front viewing field 72. In such a case, as described above, in a case where the guidance information that guides a doctor or the like to the region of interest 124 that has disappeared from the display region 115 is generated and displayed, the missed region of interest 124 is easily captured again.

Particularly, since the endoscope system 10 actually detects the region of interest 124 in the non-display region 121 and presents the direction or the like thereof without estimating a direction in which the region of interest 124 that has disappeared from the display region 115 is present, or the like, guide to the region of interest 124 that has disappeared from the display region 115 can be accurately performed.

In addition, as compared to the endoscope system 10 of the above second embodiment, the endoscope system 10 of the first embodiment is a system in which the guidance information generation unit 83generates the guidance information on the region of interest 124 detected by the region-of-interest detection unit 82 in a case where the region-of-interest detection unit 82 has detected the region of interest 124 in the non-display region 121 and in a case where the region of interest 124 is not detected in any of the display region 115 and the non-display region 121 until a predetermined time before the time when the region of interest 124 (for example, a front frame) is detected. The advantage that the guidance information is generated and displayed in the first region of interest 124 detected in the non-display region 121 is as the first embodiment. Hence, also in the endoscope system 10 of the above second embodiment, it is preferable to generate and display the guidance information on the region of interest 124 to be first detected in the non-display region 121 similarly to the first embodiment.

In the above first and second embodiments, the invention is carried out in the endoscope system 10 that performs observation by inserting the endoscope 12 provided with the image sensor 48 into the subject. However, the invention is also suitable in a capsule endoscope system. As illustrated in Fig. 20, for example, the capsule endoscope system has at least a capsule endoscope 700 and a processor device (not illustrated).

The capsule endoscope 700 comprises a light source unit 702, a control unit 703, an image sensor 704, an image processing unit 706, and a transmission/reception antenna 708. The light source unit 702 corresponds to the light source unit 20. The control unit 703 functions similarly to the light source control unit 22 and the control unit 52. Additionally, the control unit 703 is capable of wirelessly communicating with the processor device of the capsule endoscope system using the transmission/reception antenna 708. Although the processor device of a capsule endoscope system is substantially the same as that of the processor device 16 of the above embodiment, the image processing unit 706 corresponding to the image acquisition unit 54 and the image processing unit 61 is provided in the capsule endoscope 700, and the endoscope image is transmitted to the processor device via the transmission/reception antenna 708. The image sensor 704 is configured similarly to the image sensor 48.

### Explanation of References

10: endoscope system
12: endoscope
12a: insertion part
12b: operating part
12c: bending part
12d: distal end part
12e: angle knob
13: zoom operating part
14: light source device
16: processor device
18: monitor
19: console
20, 702: light source unit
22: light source control unit
30a: illumination optical system
30b: imaging optical system
41: light guide
45: illumination lens
46: objective lens
47: zoom lens
48, 704: image sensor
52, 703: control unit
54, 706: image acquisition unit
56: DSP (Digital Signal Processor)
58: noise reduction unit
59: conversion unit
61: image processing unit
66: display control unit
81: image generation unit
82: region-of-interest detection unit
83: guidance information generation unit
101: intermediate image
102: full viewing field region
103: blank region
106: front viewing field region
107: extraction range
115: display region
121: non-display region
123: center
130: arrow indicating direction of region of interest
114: display image
141: observation target
141a: fold (pleat)
143: insertion direction
700: capsule endoscope
708: transmission/reception antenna
S11 to S218: operation steps of endoscope system

## Claims

1. An endoscope system comprising:
an endoscope;
an image acquisition unit adapted to acquire an endoscope image;
a display unit adapted to display at least a portion of the endoscope image;
a region-of-interest detection unit adapted to detect a region of interest at least in a non-display region out of a display region that is a portion of the endoscope image and is to be displayed on the display unit, and the non-display region that is a portion of the endoscope image and is a portion excluding the display region from the endoscope image;
a guidance information generation unit adapted to generate guidance information to the region of interest present in the non-display region in a case where the region-of-interest detection unit has detected the region of interest in the non-display region; and
a display control unit adapted to display the guidance information on the display unit in addition to the endoscope image,
**characterized in that**
the region-of-interest detection unit is adapted to detect the region of interest in the display region,
the endoscope system further comprises a disappearance determination unit adapted to determine a disappearance of the region of interest from the display region resulting from movement of the region of interest detected in the display region at a certain time to the non-display region at a time after the certain time, and
the guidance information generation unit is adapted to generate the guidance information on the region of interest that is determined to have disappeared from the display region by the disappearance determination unit.

2. The endoscope system according to claim 1,
wherein the region of interest is a region including at least any one of a lesioned part, a benign tumor part, an inflammable part, a marking part, or a biopsy-performed part in which a biological test is performed.

3. The endoscope system according to claim 1 or 2,
wherein the guidance information generation unit is adapted to generate the guidance information including a direction of the region of interest in the non-display region.

4. The endoscope system according to any one of claims 1 to 3,
wherein the guidance information generation unit is adapted to generate the guidance information including a distance or angle to the region of interest in the non-display region.

5. The endoscope system according to any one of claims 1 to 4,
wherein the guidance information generation unit is adapted to generate the guidance information including an operation time of the endoscope taken to bring the region of interest present in the non-display region into the display region.

6. The endoscope system according to claim 5,
wherein the guidance information generation unit is adapted to calculate the operation time of the endoscope, using at least a distance from the display region to the region of interest present in the non-display region.

7. The endoscope system according to any one of claims 1 to 6,
wherein the region-of-interest detection unit is adapted to detect the region of interest present in the non-display region, using one endoscope image, and
wherein the guidance information generation unit is adapted to generate the guidance information to the region of interest present in the non-display region, using the one endoscope image.

8. The endoscope system according to any one of claims 1 to 7,
wherein the endoscope image is a wide-angle image obtained by imaging an observation target present beside or behind a distal end part of the endoscope in addition to the observation target present in front of the distal end part of the endoscope.

9. The endoscope system according to claim 8,
wherein the display region is a region including at least the observation target present in front of the distal end part of the endoscope, and the non-display region is a region including at least the observation target present beside or behind the distal end part of the endoscope.

10. A processor device comprising:
an image acquisition unit adapted to acquire an endoscope image;
a region-of-interest detection unit adapted to detect a region of interest at least in a non-display region out of a display region that is a portion of the endoscope image and is to be displayed on a display unit, and the non-display region that is a portion of the endoscope image and is a portion excluding the display region from the endoscope image;
a guidance information generation unit adapted to generate guidance information to the region of interest present in the non-display region in a case where the region-of-interest detection unit has detected the region of interest in the non-display region; and
a display control unit adapted to display the guidance information on the display unit in addition to the endoscope image,
**characterized in that**
the region-of-interest detection unit is adapted to detect the region of interest in the display region,
the endoscope system further comprises a disappearance determination unit adapted to determine a disappearance of the region of interest from the display region resulting from movement of the region of interest detected in the display region at a certain time to the non-display region at a time after the certain time, and
the guidance information generation unit is adapted to generate the guidance information on the region of interest that is determined to have disappeared from the display region by the disappearance determination unit.

## Patentansprüche

1. Endoskopsystem, umfassend:
ein Endoskop;
eine Bilderfassungseinheit, ausgebildet zum Erfassen eines Endoskopbilds;
eine Anzeigeeinheit, ausgebildet zum Anzeigen mindestens eines Abschnitts des Endoskopbilds;
eine ROI-Detektoreinheit, ausgebildet zum Nachweisen einer ROI (Region Of Interest; Bereich von Interesse) zumindest in einer anzeigefreien Zone außerhalb einer Anzeigezone, bei der es sich um einen Abschnitt des Endoskopbilds handelt, und die auf der Anzeigeeinheit anzuzeigen ist, ferner zum Nachweisen der anzeigefreien Zone, bei der es sich um einen Abschnitt des Endoskopbilds handelt, welcher Abschnitt die Anzeigezone von dem Endoskopbild ausschließt;
eine Leitinformations-Erzeugungseinheit, ausgebildet zum Erzeugen von Leitinformation hin zu der ROI, die sich in der anzeigefreien Zone befindet, für den Fall, dass die ROI-Detektoreinheit die ROI innerhalb der anzeigefreien Zone nachweist; und
eine Anzeigesteuereinheit, ausgebildet zum Anzeigen der Leitinformation auf der Anzeigeeinheit zusätzlich zu dem Endoskopbild,
**dadurch gekennzeichnet, dass**
die ROI-Detektoreinheit ausgebildet ist zum Nachweisen der ROI innerhalb der Anzeigezone,
das Endoskopsystem weiterhin eine Verschwunden-Bestimmungseinheit aufweist, die ausgebildet ist zum Bestimmen eines Verschwundenseins der ROI aus der Anzeigezone, resultierend aus der Bewegung der innerhalb der Anzeigezone nachgewiesenen ROI zu einer gewissen Zeit hin zu der anzeigefreien Zone zu einer Zeit nach der gewissen Zeit, und
die Leitinformations-Erzeugungseinheit ausgebildet ist zum Erzeugen der Leitinformation über die ROI, von der durch die Verschwundensein-Bestimmungseinheit festgestellt wurde, dass sie aus der Anzeigezone verschwunden ist.

2. Endoskopsystem nach Anspruch 1,
bei dem die ROI eine Zone ist, die einen lädierten Teil, einen benignen Tumorteil, einen entzündeten Teil, einen Markierungsteil, und/oder einen Biopsieteil, in welchem ein biologischer Test ausgeführt wird, enthält.

3. Endoskopsystem nach Anspruch 1 oder 2,
bei dem die Leitinformations-Erzeugungseinheit ausgebildet ist zum Erzeugen der Leitinformation einschließlich einer Richtung zu der ROI innerhalb der anzeigefreien Zone.

4. Endoskopsystem nach einem der Ansprüche 1 bis 3,
bei dem die Leitinformations-Erzeugungseinheit ausgebildet ist zum Erzeugen der Leitinformation einschließlich einer Distanz oder eines Winkels bezüglich der ROI innerhalb der anzeigefreien Zone.

5. Endoskopsystem nach einem der Ansprüche 1 bis 4,
bei dem die Leitinformations-Erzeugungseinheit ausgebildet ist zum Erzeugen der Leitinformation einschließlich einer Operationszeit des Endoskops, die benötigt wird, um die in der anzeigefreien Zone vorhandene ROI in die Anzeigezone zu bringen.

6. Endoskopsystem nach Anspruch 5,
bei dem die Leitinformations-Erzeugungseinheit ausgebildet ist zum Berechnen der Operationszeit des Endoskops unter Verwendung mindestens einer Distanz von der Anzeigezone zu der ROI, die sich in der anzeigefreien Zone befindet.

7. Endoskopsystem nach einem der Ansprüche 1 bis 6,
bei dem die ROI-Detektoreinheit ausgebildet ist zum Nachweisen der in der anzeigefreien Zone vorhandenen ROI unter Verwendung eines Endoskopbilds, und
wobei die Leitinformations-Erzeugungseinheit ausgebildet ist zum Erzeugen der Leitinformation zu der in der anzeigefreien Zone vorhandenen ROI unter Verwendung eines Endoskopbilds.

8. Endoskopsystem nach einem der Ansprüche 1 bis 7,
bei dem das Endoskopbild ein Weitwinkelbild ist, erhalten durch Abbilden eines Betrachtungsziels, welches sich neben oder hinter einem distalen Endteil des Endoskops befindet, zusätzlich zu dem Betrachtungsziel, welches sich vor dem distalen Endteil des Endoskops befindet.

9. Endoskopsystem nach Anspruch 8,
bei dem die Anzeigezone eine Zone ist, die mindestens das vor dem distalen Ende des Endoskops befindliche Betrachtungsziel enthält, und die anzeigefreie Zone eine Zone ist, die mindestens das Betrachtungsziel enthält, welches sich neben oder hinter dem distalen Endteil des Endoskops befindet.

10. Prozessoreinrichtung, umfassend:
eine Bilderfassungseinheit, ausgebildet zum Erfassen eines Endoskopbilds;
eine ROI-Detektoreinheit, ausgebildet zum Nachweisen einer ROI (Region Of Interest; Bereich von Interesse) zumindest in einer anzeigefreien Zone außerhalb einer Anzeigezone, bei der es sich um einen Abschnitt des Endoskopbilds handelt, und die auf der Anzeigeeinheit anzuzeigen ist, ferner zum Nachweisen der anzeigefreien Zone, bei der es sich um einen Abschnitt des Endoskopbilds handelt, welcher Abschnitt die Anzeigezone von dem Endoskopbild ausschließt;
eine Leitinformations-Erzeugungseinheit, ausgebildet zum Erzeugen von Leitinformation hin zu der ROI, die sich in der anzeigefreien Zone befindet, für den Fall, dass die ROI-Detektoreinheit die ROI innerhalb der anzeigefreien Zone nachweist; und
eine Anzeigesteuereinheit, ausgebildet zum Anzeigen der Leitinformation auf der Anzeigeeinheit zusätzlich zu dem Endoskopbild,
**dadurch gekennzeichnet, dass**
die ROI-Detektoreinheit ausgebildet ist zum Nachweisen der ROI innerhalb der Anzeigezone,
das Endoskopsystem weiterhin eine Verschwunden-Bestimmungseinheit aufweist, die ausgebildet ist zum Bestimmen eines Verschwundenseins der ROI aus der Anzeigezone, resultierend aus der Bewegung der innerhalb der Anzeigezone nachgewiesenen ROI zu einer gewissen Zeit hin zu der anzeigefreien Zone zu einer Zeit nach der gewissen Zeit, und
die Leitinformations-Erzeugungseinheit ausgebildet ist zum Erzeugen der Leitinformation über die ROI, von der durch die Verschwundensein-Bestimmungseinheit festgestellt wurde, dass sie aus der Anzeigezone verschwunden ist.

## Revendications

1. Système d'endoscope, comprenant :
un endoscope ;
une unité d'acquisition d'image, apte à acquérir une image d'endoscope ;
une unité d'affichage, apte à afficher au moins une portion de l'image d'endoscope ;
une unité de détection de région d'intérêt, apte à détecter une région d'intérêt au moins dans une région sans affichage dans une région d'affichage, laquelle est une portion de l'image d'endoscope et doit être affichée sur l'unité d'affichage, et la région sans affichage, laquelle est une portion de l'image d'endoscope et est une portion excluant de l'image d'endoscope la région d'affichage ;
une unité de génération d'informations de guidage, apte à générer des informations de guidage vers la région d'intérêt présente dans la région sans affichage dans un cas où l'unité de détection de région d'intérêt a détecté la région d'intérêt dans la région sans affichage, et
une unité de commande d'affichage, apte à afficher les informations de guidage sur l'unité d'affichage en plus de l'image d'endoscope,
**caractérisé en ce que**
l'unité de détection de région d'intérêt est apte à détecter la région d'intérêt dans la région d'affichage ;
le système d'endoscope comprend en outre une unité de détermination de disparition, apte à déterminer une disparition de la région d'intérêt dans la région d'affichage, résultant du déplacement de la région d'intérêt détectée dans la région d'affichage à un certain temps vers la région sans affichage à un temps après le certain temps, et
l'unité de génération d'informations de guidage est apte à générer les informations de guidage sur la région d'intérêt, laquelle est déterminée comme ayant disparu de la région d'intérêt par l'unité de détermination de disparition.

2. Système d'endoscope selon la revendication 1,
dans lequel la région d'intérêt est une région incluant au moins une quelconque partie parmi une partie présentant une lésion, une partie de tumeur bénigne, une partie inflammatoire, une partie de marquage, ou une partie de réalisation de biopsie, où un essai biologique est réalisé.

3. Système d'endoscope selon la revendication 1 ou 2,
dans lequel l'unité de génération d'informations de guidage est apte à générer les informations de guidage incluant une direction de la région d'intérêt dans la région sans affichage.

4. Système d'endoscope selon l'une quelconque des revendications 1 à 3,
dans lequel l'unité de génération d'informations de guidage est apte à générer les informations de guidage incluant une distance ou un angle par rapport à la région d'intérêt dans la région sans affichage.

5. Système d'endoscope selon l'une quelconque des revendications 1 à 4,
dans lequel l'unité de génération d'informations de guidage est apte à générer les informations de guidage incluant un temps de fonctionnement de l'endoscope pris pour amener la région d'intérêt présente dans la région sans affichage dans la région d'affichage.

6. Système d'endoscope selon la revendication 5,
dans lequel l'unité de génération d'informations de guidage est apte à calculer le temps de fonctionnement de l'endoscope, à l'aide d'au moins une distance allant de la région d'affichage jusqu'à la région d'intérêt présente dans la région sans affichage.

7. Système d'endoscope selon l'une quelconque des revendications 1 à 6,
dans lequel l'unité de détection de région d'intérêt est apte à détecter la région d'intérêt présente dans la région sans affichage à l'aide d'une image d'endoscope, et
dans lequel l'unité de génération d'informations de guidage est apte à générer les informations de guidage vers la région d'intérêt présente dans la région sans affichage, à l'aide de la une image d'endoscope.

8. Système d'endoscope selon l'une quelconque des revendications 1 à 7,
dans lequel l'image d'endoscope est une image grand-angulaire obtenue en imageant une cible d'observation présente à côté ou derrière une partie d'extrémité distale de l'endoscope outre la cible d'observation présente devant la partie d'extrémité distale de l'endoscope.

9. Système d'endoscope selon la revendication 8,
dans lequel la région d'affichage est une région incluant au moins la cible d'observation présente devant la partie d'extrémité distale de l'endoscope, et la région sans affichage est une région incluant au moins la cible d'observation présente à côté ou derrière la partie d'extrémité distale de l'endoscope.

10. Dispositif de processeur, comprenant :
une unité d'acquisition d'image, apte à acquérir une image d'endoscope ;
une unité de détection de région d'intérêt, apte à détecter une région d'intérêt au moins dans une région sans affichage dans une région d'affichage, laquelle est une portion de l'image d'endoscope et doit être affichée sur l'unité d'affichage, et la région sans affichage, laquelle est une portion de l'image d'endoscope et est une portion excluant de l'image d'endoscope la région d'affichage ;
une unité de génération d'informations de guidage, apte à générer des informations de guidage vers la région d'intérêt présente dans la région sans affichage dans un cas où l'unité de détection de région d'intérêt a détecté la région d'intérêt dans la région sans affichage, et
une unité de commande d'affichage, apte à afficher les informations de guidage sur l'unité d'affichage en plus de l'image d'endoscope,
**caractérisé en ce que**
l'unité de détection de région d'intérêt est apte à détecter la région d'intérêt dans la région d'affichage ;
le système d'endoscope comprend en outre une unité de détermination de disparition, apte à déterminer une disparition de la région d'intérêt dans la région d'affichage résultant du déplacement de la région d'intérêt détectée dans la région d'affichage à un certain temps vers la région sans affichage à un temps après le certain temps, et
l'unité de génération d'informations de guidage est apte à générer les informations de guidage sur la région d'intérêt, laquelle est déterminée comme ayant disparu de la région d'intérêt par l'unité de détermination de disparition.
